## Europäisches Patentamt

## European Patent Office

## Office européen des brevets

(19)

(11) Veröffentlichungsnummer: **0 170 880**
**B1**

(12) **EUROPÄISCHE PATENTSCHRIFT**

(45) Veröffentlichungstag der Patentschrift: **06.02.91**

(21) Anmeldenummer: **85108206.5**

(22) Anmeldetag: **03.07.85**

(51) Int. Cl.⁵: **C 12 N 9/06**, C 12 P 39/00, A 23 L 1/015, C 12 H 1/00, A 23 K 1/00

(54) **Verfahren zur Herstellung von Aminooxydase enthaltendem Material, bzw. Aminooxydase, aus Mikroorganismen, solche Produkte und deren Verwendung zum Abbau von Histamin in Nahrungsmitteln, Getränken und Futtermitteln.**

(30) Priorität: **09.07.84 CH 3321/84**

(43) Veröffentlichungstag der Anmeldung:
**12.02.86 Patentblatt 86/07**

(45) Bekanntmachung des Hinweises auf die Patenterteilung:
**06.02.91 Patentblatt 91/06**

(84) Benannte Vertragsstaaten:
**AT BE CH DE FR GB IT LI LU NL SE**

(56) Entgegenhaltungen:
EP-A-0 132 674
DE-A-3 206 826
FR-A-2 101 095
US-A-2 289 194

BIOCHEM. J., Band 211, Nr. 2, 1983, Seiten 481-493, GB; J. GREEN et al.: "Serological differences between the multiple amine oxidases of yeasts and comparison of the specificities of the purified enzymes from Candida utilis and Pichia pastoris"

(73) Patentinhaber: **Underberg, Emil**
**Industriestrasse 31**
**CH-8305 Dietlikon (CH)**

(72) Erfinder: **Underberg, Emil**
**Industriestrasse 31**
**CH-8305 Dietlikon (CH)**
Erfinder: **Lembke, Andreas**
**Eutiner Landstrasse 15**
**D-2420 Eutin-Sielbeck (DE)**

(74) Vertreter: **Goddar, Heinz J., Dr. et al**
**FORRESTER & BOEHMERT Widenmayerstrasse 4/I**
**D-8000 München 22 (DE)**

(56) Entgegenhaltungen:
BIOCHEM. J., Band 199, Nr. 1, 1981, Seiten 187-201, GB; G.W. HAYWOOD et al.: "Microbial oxidation of amines. Distribution, purification and properties of two primary-amine oxidases from the yeast Candida biodinii grown on amines as sole nitrogen source"

Courier Press, Leamington Spa, England.

(56) Entgegenhaltungen:

CHEMICAL ABSTRACTS, Band 63, Nr. 4, 16. August 1965, Nr. 4587e,f Columbus, Ohio, US; HIDEAKI YAMADA et al.: "Crystalline amine oxidase of Aspergillus niger"

CHEMICAL ABSTRACTS, Band 97, Nr. 9, 30. August 1982, Seite 326, Nr. 69040h, Columbus, Ohio, US; J. GREEN et al.: "More than one amine oxidase is involved in the metabolism by yeasts of primary amines supplied as nitrogen source"

**Beschreibung**

Es ist bekannt, dass eine grosse Anzahl an Nahrungsmitteln, Futtermitteln und Getränken einen Gehalt an Histamin aufweisen. Insbesondere handelt es sich bei diesen Produkten um solche, bei deren Herstellung eine durch Mikroorganismen hervorgerufene Umwandlung durchgeführt wurde. Als Beispiele hiefür seien Produkte erwäht, die durch eine alkoholische Gärung gewonnen werden, wie zum Beispiel Wein, Schaumwein, Bier und gebrannte Alkoholika und ferner Produkte, bei deren Herstellung eine Milchsäuregärung durchgeführt wurde, wie zum Beispiel Sauermilchprodukte, wie Joghurt, Sauermilch und Quark, sowie auch pflanzliche Produkte, die einer Milchsäuregärung unterworfen worden waren, wie zum Beispiel saure Gurken, Sauerkraut und Silofutter. Dazu gehören ferne auch verschiedene Käsesorten, bei deren Herstellung aus einem mit Labferment gewonnenen Milchkoagulat oder Quark verschiedene Gärungen nebeneinander ablaufen, darunter auch Milchsäuregärungen.

Der Grund für den relativ hohen Histamingehalt dieser Produkte ist darin zu sehen, dass in der natürlich vorkommenden Mikroorganismenpopulation vorwiegend solche Mikroorganismen enthalten sind, die neben der erwünschten Umwandlung, also einer alkoholischen Gärung oder einer Milchsäuregärung, noch das im Ausgangsmaterial enthaltene Histidin unter Bildung von Histamin abbauen. Wird ein histaminhaltiges Silofutter verfüttert, dann gelangt das den Kühen zugeführte Histamin auch in die Milch.

Histamin ist bekanntlich für viele Personen schädlich, insbesondere Allergiker.

Ziel der vorliegenden Erfindung war es, ein Aminooxydase enthaltendes Material, bzw. Aminooxydase, auf mikrobiologischem Wege herzustellen.

Ein weiteres Ziel der vorliegenden Erfindung war es, dieses Aminooxydase enthaltendes Material, bzw. die Aminooxydase zu verwenden, um durch einen enzymatischen Abbau Histamin aus Nahrungsmitteln, Getränken und Futtermitteln zu entfernen.

Stand der Technik

In der GB—A—2 031 948 wurde bereits ein Verfahren zur Herstellung von Nahrungsmitteln, Genussmitteln, Getränken und Futtermitteln beschrieben, die einen verminderten Histamingehalt besitzen oder überhaupt Histaminfrei sind. Dieses Verfahren beruht darauf, dass unter der natürlich vorkommenden Mikroorganismenpopulation und auch unter den bei verschiedenen Zellkultursammlungen schon seit langem hinterlegten Stämme, auch solche vorhanden sind, die das im Ausgangsmaterial enthaltene Histidin ohne Bildung von Histamin abbauen. Bei dem in der erwähnten britischen Patentschrift bechriebenen Verfahren werden gezielt solche Einzelkulturen zur Herstellung der Nahrungsmittel, Getränke und Futtermittel eingesetzt, die Histidin ohne Bildung von Histamin abbauen. Dabei wird entweder die gewünschte Umwandlung, beispielsweise eine alkoholische Gärung oder Milchsäuregärung, bezielt mit solchen kein Histamin bildenden Stämmen durchgeführt oder es wird bei Produkten mit nur geringem Gehalt an Eiweissstoffen, beispielsweise bei der Durchführung einer alkoholischen Gärung, zunächst aus dem Ausgangsmaterial das vorhandene Histidin mit solchen Mikroorganismenstämmen abgebaut, die daraus kein Histamin bilden, und das so gewonnene histidinfreie Ausgangsmaterial wird dann der weiteren Umwandlung unter Verwendung beliebiger Mikroorganismenstämme unterworfen, also auch solcher, die vorhandenes Histidin in Histamin abbauen würden. So kann beispielsweise aus dem sterilisierten Most zunächst mit einem kein Histamin bildenden Lactobazillusstamm das Histidin abgebaut werden und anschliessend kann dieses histidinfreie Produkt, wie üblich, mit beliebigen Hefen zu einem Wein vergoren werden. Statt der Verwendung eines Mikroorganismuses zum Abbau des Histidines ohne Bildung von Histamin, kann auch ein aus einem derartigen Mikroorganismenstamm gewonnenes Enzym eingesetzt werden. Das in dieser britischen Patentschrift beschrieb- ene Verfahren besitzt jedoch den Nachteil, dass es nicht möglich ist, mit diesem Verfahren bereits vorhandenes Histamin abzubauen, sondern es kann so nur die Bildug von Histamin aus Histidin verhindert werden. Wenn beispielsweise eine Milch infolge der Verfütterung ovn histaminhaltigem Silofutter bereits eine gewisse Menge an Histamin enthält, dann kann bei der Durchführung einer Milchsäuregärung unter gezieltem Einsatz von solchen Milchsäure bildenden Mikroorganismen, die aus Histidin kein Histamin bilden, nur verhindert werden, dass das so erhaltene Sauermilchprodukt einen höheren Gehalt an Histamin aufweist als die zu dessen Herstellung verwendete Frischmilch. Das bereits in der Frischmilch vorhandene Histamin wird durch die eingesetzten Mikroorganismenstämme jedoch nicht zerstört.

Es ist ferner bekannt, dass mit Hilfe von Histaminasen, d.h. mit Histamin abbauenden Enzymen, Histamin abgebaut werden kann.

Histaminasen sind im Blutserum von Säugetieren, beispielsweise im Serum von Schweinen, vorhanden und Histaminasen sind auch in verschiedenen Geweben von Säugetieren zu finden.

Histaminasen, nämlich D(-)-Aminooxydasen wurden beispielsweise in den Peroxysomen, also den sogenannten Mikrobodies, der Leber festgestellt und in diesen Peroxysomen sind ferner auch Katalasen vorhanden, die den Abbau des durch Oxydasen gebildeten Wasserstoffperoxides bewirken.

In der USA Patentschrift Nr. 2 289 194 wird beispielsweise ein Verfahren zur Herstellung eines beständigen Histaminasepräparates, ausgehend von Schweinenieren, beschrieben, wobei bei diesem Verfahren fein zerkleinerte Nieren verschiedenen Extraktionsverfahren und Fällungsverfahren unterworfen werden.

In der FR—A—2 101 095 wird ferner ein Verfahren zur Herstellung von Diamin-Oxydase enthaltenden Materialien beschrieben, bei dem als Ausgangsmaterial für die Gewinnung dieses Enzymproduktes menschliche Placenta verwendet wird.

Alle diese bisher bekannten Verfahren, bei denen Histaminase oder ein Histaminase enthaltendes Produkt aus Geweben von Säugetieren oder dem Blutserum von Säugetieren isoliert werden, besitzen jedoch den Nachteil, dass die Isolierung ziemlich kompliziert ist und üblicherweise viele Reinigungsschritte umfasst, wie zum Beispiel Dialysen, mehrere Extraktionen, Fällungen und ähnliche Verfahrensschritte. Häufig sind auch die entsprechenden Gewebe oder Organe nicht in ausreichendem Ausmass vorhanden, damit daraus genügende Mengen an Hisaminase hergestellt werden können, beziehungsweise auch oft nur zu relativ hohem Preise erhältlich. Es ist ferner bekannt, dass Histaminasen auch in Mikroorganismen vorkommen.

In der Veröffentlichung aus "Chemical Abstracts", Band 99, Nr. 3, Seite 343, Abstract 19342d vom 18. Juli 1983, werden die serologischen Unterschiede zwischen Aminooxydasen der Hefe beschrieben. Ferner werden die gereinigten Enzyme miteinander verglichen, die aus dem Mikroorganismus Candida utilis und dem Mikroorganismus Pichia pastoris isoliert wurden. Auch die Oxydation einiger Amine und Aminosäuren, so beispielsweise Lysin und Ornithin, mit Hilfe dieser Aminooxydasen wurden untersucht. Aus diesem Abstract ist jedoch nicht zu ersehen, ob die fraglichen Aminooxydasen Histamin abbauen oder nicht.

In dem "Chemical Abstracts", Band 96, Nr. 13, Seite 382, Abstract 100569t vom 29. März 1982 wird die von Mikroorganismen hervorgerufene Oxydation von Aminen untersucht. Die Eigenschaften von zwei Oxydasen für primäre Amine, die aus dem Mikroorganismenstamm Candida boidinii isoliert wurden, wurden getestet, jedoch kann dieser Veröffentlichung ebenfalls kein Hinweis entnommen werden, ob die fragliche Aminooxydase Histamin abbaut oder nicht.

In der DE—A—3 206 826 wird ein Verfahren zur Herstellung von Aminooxydase durch Züchtung von Aminooxydase bildenden Mikroorganismen beschrieben. Zur Durchführung dieses Verfahrens werden ausschliesslich Mikroorganismen der folgenden Klassen von Mikroorganismen eingesetzt: Talaromyces, Eupenicillium, Petromyces, Neosartorya und Eurotium.

In der EP—A2—0132674 ist ein Verfahren beschrieben, bei dem Aspergillus niger zur Herstellung eines Aminooxydasepräparates eingesetzt wird.

Die Erfindung betrifft ein Verfahren zum Abbau von Histamin in Nahrungsmitteln, Getränken und/ oder Futtermitteln durch Zusatz eines aus Mikroorganismen gewonnenen D(-)-Aminooxydasepräparates.

Aufgabe der Erfindung ist es, ein Verfahren der eingangs genannten Art so auszugestalten, daß Präparat in größeren Mengen und wirtschaftlich hergestellt werden kann.

Die Erfindung ist dadurch gekennzeichnet, daß als Mikroorganismus ein Lactobacillus eingesetzt wird, der aus Glucose, anderen Zuckern oder anderen Kohlehydraten homofermentativ Milchsäure bildet, und das D(-)- Aminooxydasepräparat mindestens Teile der vom Zuchtmedium befreiten, zerkleinerten Mikroorganismen enthält.

Bevorzugt wird als Mikroorganismus eingesetzt: Lactobacillus delbrückii, Lactobacillus leichmanii oder Lactobacillus bulgaricus, weil diese Mikroorganismen, wie langjährige praktische Beobachtungen bestätigen, keinerlei toxische Stoffe produzieren.

Die eingesetzten Mikroorganismen werden D(-)-aminooxydasebildend gezüchtet. Die in dem Präparat enthaltene D(-)-Aminooxydase ist eine Histaminase, die in Gegenwart von Sauerstoff besonders günstig bei pH-Werten im Bereich von 6,7 bis 7,6 Histamin unter Bildung von Ammoniak, Wasserstoffperoxid und Aminoaldehyden oxydiert.

Die bei Durchführung des erfindungsgemäßen Verfahrens gebildete Histaminase verbleibt während der Zucht zum größten Teil in den Mikroorganismen selbst, wird also nicht oder nur in geringen Mengen während der Zucht an das Kulturmedium abgegeben. Bei der Durchführung des erfindungsgemäßen Verfahrens kann also nach der Abtrennung der Mikroorganismen aus dem Zuchtmedium das Zuchtmedium verworfen werden und nur die daraus isolierten Mikroorganismen müssen weiter aufgearbeitet werden. Zur Gewinnung der Histaminase aus den Zellen der Mikroorganismen ist ferner eine Zerkleinerung der Zellen also ein Zellaufschluss, erforderlich, damit die Histaminase aus den Zellen austreten kann und mit deren Hilfe der Abbau des Histamines durchgeführt werden kann.

Verwendet man nach dem erfindungsgemässen Verfahren zur Herstellung des Histaminase enthaltenden Materiales bestimmte Milchsäurebakterien, beispielsweise Mutanten von Lactobacillus delbrueckii oder Lactobacillus leichmannii, die beide aus Glucose oder anderen Kohlehydraten homofermentativ D(-)-Milchsäure bilden, dann muss man grosse Mengen an Zellen züchten, weil die fraglichen Mikroorganismen neben der Histaminase auch noch Transaminasen und Desaminasen bilden.

Gemäss einer weiteren Ausführungsart des erdingungsgemässen Verfahrens, wird zur Herstellung des Histaminase enthaltenden Materiales eine Mischkultur aus mindestens einer Lactobazillusspezies und Candidacrusei gezüchtet. Dabei hält man vorzugsweise solche Züchtungsbedingungen ein, bzw. wählt solche Mikroorganismenstämme aus, dass der Lactobazillus im Substrat enthaltene Zucker unter Bildung von Milchsäure abbaut, während die eingesetzte Candidacrusei Species die so gebildete Milchsäure abaut, jedoch die im Nährmedium vorhandenen Zucker nicht angreift. Durch diese Arbeitsweise werden die im Nährmedium enthaltenen Zucker zunächst von den Lactobacillen zu Milchsäure abgebaut und diese wird dann als Kohlenstoffquelle von der Hefe verwendet und weiter abgebaut. Ferner wird der durch die Histaminase gebildete Ammoniak von der Hefe für die Proteinsythese genutzt.

Wesentlich bei der Verwendung einer Mischkultur aus mindestens einer Lactobacillusspezies und Candidacrusei zur Herstellung des erfindungsgemässen Histaminase enthaltenden Materiales ist es dabei, die fraglichen Mikroorganismenstämme so auszuwählen, dass ihre optimalen Wachstumsbedingungen möglichst ähnlich sind, nämlich beispielsweise die optimale Wachstumstemperatur, der pH-Wert des Kulturmediums und ähnliches.

Eine speziell bevorzugte Mischkultur aus einer Lactobacillusspezies und einer Candidaspezies, die zur Herstellung des erfindungsgemässen, Histaminase enthaltenden Materials angewandt wird, ist eine Mischkultur aus Lactobacillus bulgaricus und Candida crusei.

Ein bevorzugtes Zuchtemdium, das bei der Durchführung des erfindungsgemässen Verfahrens zur Züchtung der Mikroorganismen herangezogen wird, ist ein Milchzucker enthaltendes Medium. Das fragliche Medium erwies sich als speziell vorteilhaft für die Züchtung einer Mischkultur aus mindestens einer Lactobacillusspezies und Candidacrusei. Aus Gründen der Wirschaftlichkeit wird als Milchzucker enthaltendes Zuchtmedium besonders vorteilhaft Molke verwendet, insbesondere eine solche Molke, deren Eiweissgehalt vor der Verwendung gesenkt wurde oder die vollständig vom Eiweiss befreit wurde.

Zu Beginn der Gärung setzt man der Molke ein Hefeautolysat zu.

Gemäss einer bevorzugten Ausführungsart dieser Mischkulturzüchtung wird in zwei Fermentern gearbeitet. Nach der Anfangsphase wird dem ersten Fermenter die Molke mit konstanter Zuflussgeschwindigkeit zugeführt und in diesem ersten Fermenter wachsen die dort befindlichen Milchsäurebakterien und Hefen bei begrenzter Luftzufuhr. Dadurch wird der Milchzucker der Molke restlos in Milchsäure umgewandelt. Zur Einleitung ihres Wachstums nehmen die Hefen im ersten Fermenter vorwiegend Milchsäure aus dem Medium auf und erst in der nachfolgenden Wachstumsphase, die in einem zweiten Fermenter durchgeführt wird, wird der Ammoniak assimiliert. Das Wachstum der Hefen, das mit der Stickstoffaufnahme verbunden ist, reicht im ersten Fermenter im allgemeinen aus, um die Zusammensetzung der Mischkultur konstant zu halten.

Der pH-Wert des Mediums im ersten Fermenter wird laufend gemessen und durch Zugabe von Ammoniak konstant gehalten.

Auch im zweiten Fermenter wird dauernd der pH-Wert gemessen und konstant gehalten, und zwar indirekt, indem man die Luftzufuhr im ersten Fermenter entsprechend regelt.

Durch die hier beschriebene Arbeitsweise wird bei der Mischkultur im Zuchtmedium ein optimales Verhältnis der intermediär gebildeten Produkte Ammoniumlactat zu milchsäure eingestellt und diese Kohlenstoffquelle wird dabei ständig von der Hefe abgebaut. Der zweite Fermenter wird im Gegensatz zum ersten Fermenter optimal belüftet.

Bei dieser Züchtung in der Mischkultur wird die von den Milchsäurebakterien gebildete Milchsäure ständig von der anwesenden Hefe abgbaut, sodass der Milchzucker durch die Milchsäurebakterien quantitativ in Milchsäure umgewandelt wird. Am Ende der Züchtung ist in dem Züchtungsmedium im allgemeinen Milchzucker überhaupt nicht mehr nachweisbar, insbesondere dann, wenn die Züchtung in kontinuierlicher Kultur vorgenommen wurde.

Bei der Durchführung des erfindungsgemässen Verfahrens werden, wie bereits erwähnt, vorzugsweise ein oder mehrere Mikroorganismenstämme gezüchtet, die aus den folgenden Klassen von Mikroorganismen ausgewählt werden: Lactobacillus delbrueckii, Lactobacillus leichmannii, Lactobacillus bulgaricus und Candida crusei. Wenn man das erfindungsgemässe Verfahren durch Züchtung einer Mischkultur aus einer Lactobacillusspezies und Candida crusei durchführt, dann ist eine speziell bevorzugte Mischkulture diejenige aus Lactobacillus bulgarious und Candida crusei. In diesem Falle soll die Temperatur im Fermenter im Bereich von 42—56°C, vorzugsweise bei etwa 44°C eingehalten werden und der pH-Wert im Fermenter im Bereich von 5,0—5,5 konstant gehalten werden.

Unabhängig davon, welche speziellen Mikroorganismen zur Durchführung des erfindungsgemässen Verfahrens eingesetzt wurden und in welchem speziellen Medium die Züchtung der Mikroorganismen vorgenommen wurde, werden dann, sobald eine ausreichende Menge an Mikroorganismen herangewachsen ist, diese aus dem Zuchtmedium abgetrennt, von anhaftenden Mengen des Zuchtmediums durch Waschen oder erneutes Suspendieren in einem flüssigen Medium und erneute Abtrennung befreit und anschliessend werden dann die Mikroorganismenzellen zerkleinert.

Gemäss einer Ausführungsart des erfindungsgemässen Verfahrens wird aus den so erhaltenen zerkleinerten Mikroorganismen direkt ein lagerfähiges D(-)-Aminoxydase enthaltendes Enzympräparat hergestellt, indem man die zerkleinerten Mikroorganismen im Vakuum trocknet, wobei vorzugsweise die Trocknung bei einer Temperatur von weniger als 7°C vorgenommen wird. Vorzugsweise wird die Trocknung bei einer Temperatur von +4°C vorgenommen.

Das in der Folge beschriebene Arbeitsverfahren hat sich als vorteilhaft zur einfachen Isolierung der Mikroorganismen aus dem Zuchtmedium und zur Gewinnung des lagerfähigen Histaminase enthaltenden Enzymproduktes erwiesen.

Die im Zuchtmedium enthaltenen Mikrooganismenzellen werden durch Zentrifugieren, beispielsweise bei 15'000 bis 25'000 g abgeschleudet und die so erhaltene Biomasse wird auf +4°C gekühlt. Anschliessend wird ein Gewichtsteil dieser abgeschleuderten Biomasse mit 8 bis 12 Gewichtsteilen physiologischer Kochsalzlösung zusammengebracht und die Mikroorganismeuzellen werden in dieser Lösung erneut suspendiert. Anschliessend werden die Mikroorganismeuzellen wieder durch Zentrifugieren abgeschieden, vorzugsweise unter den gleichen Bedingungen, die zur Isolierung der Mikroorganismenzellen aus dem Zuchtmedium angewandt wurden. Die so gereinigten Mikroorganismen werden dann zekleinert und die erhaltene Biomasse im Vakuum getrocknet, beispielsweise lyophilisiert. Man erhält so ein Histaminase enthaltendes haltbares rohes Enzymprodukt. Dieses Rohprodukt ist in der Lage, nach Einbringung in einen 0,2 molaren Acetat-Puffer eines pH-Wertes von 4,9 Histamin abzubauen.

Gemäss einer anderen Arbeitsweise erfolgt zunächst nach dem oben beschriebenen Verfahren die Isolierung der Mikroorganismenzellen aus dem Zuchtmedium und deren erneute Suspension in physiologischer Kochsalzlösung und nachfolgende Abtennung durch Zentrifugieren. Die so gereinigten Mikroorganismenzellen werden dan zerkleinert und die erhaltene Biomasse wird mit der 3- bis 6-fachen Menge an kaltem Aceton versetzt und unter Kühlung so lange gerührt, bis eine Gerinnung eintritt und sich ein Sediment abscheidet. In diesem Arbeitsverfahen wird vorzugsweise bei einer Temperatur von weniger als +7°C, beispielsweise bei etwa 4°C gearbeitet.

Anschleissend wird die über dem Sediment stehende Flüssigkeit entfernt, der Rückstand filtriert und der Filterkuchen sorgfältig gewaschen, zweckmässigerweise zunächst mit einem mit Wasser mischbaren organischen Lösungsmittel, das leicht verdampft, beispielsweise mit Aceton, und anschliessend erneut mit einem leicht abdampfbaren organischen Lösungsmittel, beispielsweise mit Diäthyläther. Nach dem letzten Waschvorgang wird erneut abgesaugt und das Produkt ohne Erwärmung getrocknet, beispielsweise zunächst an der Luft vorgetrocknet und dann im Vakuum über einem Trocknungsmittel, beispielsweise Schwefelsäure nachgetrocknet. Für die Haltbarkeit des Produktes ist es wesentlich, dass es sorgsam getrocknet wurde und dass es unter trockenen Bedingungen, vorzugsweise einer Temperatur von nicht über 7°C aufbewahrt wird.

Gemäss einer weiteren Ausführungsart des Isolierverfahrens werden die im Zuchtmedium enthaltenen Mikroorganismenzellen durch Zentrifugieren abgeschleudert. Anschliessend wird die abgeschleuderte Biomasse wieder mit einer physiologischen Kochsalzlösung zusammengebracht und darin suspendiert und anschliessend werden die Mikroorganismenzellen wieder durch Zentrifugieren abgeschieden. Diese Biomasse wird dann bei einer Temperatur von weniger als 7°C, vorzugsweise bei +4°C zerkleinert, beispielsweise unter Verwendung eines Homogenisators. Wesentlich ist, dass die Zellen möglichst fein zerkleinert werden.

Anschliessend können die zerkleinerten Zellen mit einem Extraktionsmittel behandelt werden, beispielsweise mit ener 0,85 Gew.-%-igen Natriumchloridlösung, wobei man so eine Rohenzymlösung erhält.

Gemäss einer anderen Arbeitsweise wird aus den zerkleinerten Zellen ein Press-Saft abgepresst und dieser wird in einer eisgekühlten Vorlage sofort mit Amonsulfat in der Kälte, vorzugsweise bei 5°C bis zur Erreichung einer Konzentration von 25 Gew.-% an Ammoniumsulfat versetzt. Dabei bildet sich ein Niederschlag, der abzentrifugiert und verworfen wird. Die überstehende Flüssigkeit wird dann, ebenfalls in gekühlten Zustand, vorzugsweise bei +5°C, mit weiteren Mengen an Ammonsulfat versetzt, bis in der Flüssigkeit ein Gehalt von 55 Gew.-% an Ammonsulfat erreicht ist. Dabei bildet sich ein die Histaminase enthaltendes Sediment. Dieses wird abzentrifugiert, im Vakuum unter Verwendung eines Trocknungsmittels getrocknet, gemahlen und als atrokkenes Enzympulver aufbewahrt.

Aus der nach dem obigen Verfahren hergestellten Roh-Enzymlösung kann man die einzelnen Enzyme mit Hilfe einer Säulenchromatographie abtrennen. Geeignete Säulenfüllengen sind Sephadex des G-Typs, wie zum Beispiel Sephadex® G-25, sowie ferner DEAE-Sephadex® A 50. Auch Säulenfüllungen, die Kationenaustauscher sind, wie zum Beispiel Carboxymethylcellulose, sind gut geeignet.

Gemäss einer möglichen Arbeitsweise wird die Roh-Enzymlösung zunächst durch eine Säulegeleitet, die mit Sephadex® G-25 gefüllt ist. Nach Durchlauf durch diese Säule erhält man ein von Salzen freies Fermentpräparat. Für die weitere Reinigung dieses salzfreien Fermentpräparates ist sowohl eine mit DEAE-Sephadex® A-50 gefüllte Säule als auch eine mit Carboxymethylcellulose gefüllte Säule geeignet.

Arbeitet man mit einer mit Carboxymethylcellulose gefüllten Säule, so ist es zweckmässig, die Säulenfüllung zunächst mit einer geeigneten Pufferlösung zu äquilibrieren, wobei Sorge getragen werden soll, dass der pH-Wert der Pufferlösung um 0,5— 1 pH-Stufen saurer liegt als der isoelektrische Punkt der zu trennenden Protein.

Nach dem Aequilibrieren der mit Carboxymethylcellulose gefüllten Säule wird dan die Enzymlösung aufgetragen. Die Elution erfolgt dann mit Puffern, die einen höheren pH-Wert besitzen als der zur Aequilibrierung verwendete Puffer. Entweder verwendet man zur Elution nacheinander Puffer mit jeweils steigendem pH-Wert oder man verwendet als Elutionsmittel einen Puffer mit gleichbleibendem pH-Wert, wobei jedoch der Salzgradient des Elutionsmittels nach und nach gesteigert wird. Als Puffer sind beliebige anionische Puffer geeignet, wie zum Beispiel Acetatpuffer oder Phosphatpuffer.

Ein weiterer Gegenstand der vorliegenden Erfindung ist ein nach dem erfindungsgemäßen Verfahren hergestelltes D-(-)-Aminooxydasepräparat zum Abbau von Histamin in Nahrungsmitteln, Getränken und/oder Futtermitteln. Das Präparat ist dadurch gekennzeichnet, daß es aus einem Lactobacillus gewonnen ist und die D-(-)-Aminooxydase und mindestens Teile der vom Zuchtmedium befreiten, zerkleinerten Mikroorganismen enthält.

Das Enzym findet sich innerhalb der Zellen, so daß das Zuchtmedium ausgewaschen werden kann, ohne daß dadurch der Enzymgehalt beeinträchtigt wird. Nach dem Zerkleinern der Zellen ist das Enzym im Präparat verfügbar, und zwar in vorteilhafter Weise zusammen mit dem zwangsläufig mitgebildeten C-Enzym. Der zerkleinerte Mikroorganismus ist nicht mehr vermehrungsfähig aber noch enzymaktiv.

Bevorzugt werden die Nahrungsmittel oder Futtermittel in flüssigem Zustand mit dem Diaminoxidase enthaltenden oder aus Diaminoxidase bestehenden Präparat behandelt. Zu diesem Zweck versetzt man die flüssigen Nahrungsmittel oder Futtermittel mit dem Histaminase enthaltenden oder aus Histaminase bestehenden Präparat. Nach einiger Zeit wird dann mit den in der Literatur beschriebenen kolorimetrischen Testverfahren geprüft, ob das füssige Nahrungmittel frei von Histamin ist. Gegebenenfalls kann anschliessend die Histaminase durch Erhitzen der flüssigen Nahrungsmittel oder Futtermittel desaktikviert werden. Im allgemeinen ist jedoch ein derartiger Erhitzungsschritt nicht erforderlich, weil normalerweise nach einiger Zeit die Histaminase in der Mischung mit dem flüssigen Nahrungsmittel oder Futtermittel ohne weitere Behandlungen von selbst desaktiviert wird.

Es ist ferner möglich, aus flüssigen Nahrungsmitteln oder Futtermitteln, die unter Verwendung des Histaminase enthaltenden oder aus Histaminase bestehenden Produktes von Histamin befreit wurden, anschliessend feste Nahrungsmittel oder Futtermittel herzustellen.

Wie bereits erwähnt wurde, enthält Frischmilch häufig schon Histamin, und zwar aufgrund dessen, dass histaminhaltiges Silofutter an die Kühe verfüttert wurde und das Histamin dann in die Milch überging. Man kann das Histamin solcher Frischmilch entfernen, indem man sie mit den nach den erfindungsgemässen Verfahren hergestellten Histaminase enthaltenden oder aus Histaminase bestehenden Produkten zusammenbringt, beispielsweise bei einem pH-Wert, der bei etwa neutral liegt. Anschliessend kann man dann aus dieser Milch in üblicher Weise durch Zugabe von Labferment das Milchkoagulat herstellen und dieses beispeilsweise zu Käsen weiter verarbeiten. Sowohl das Milchkoagulat als auch die zurückbleibende Molke wird dabei in histaminfreiem Zustand gewonnen, was insbesondere dann von Bedeutung ist, wenn aus der Molke ein Molkengetränk hergestellt werden soll.

Die nach dem erfindungsgemässen Verfahren hergestellten Histaminase enthaltenden oder aus Histaminase bestehenden Produkte können jedoch das Histamin auch bei einem im sauren Bereich liegenden pH-Wert abbauen, beispielsweise bei pH-Werten im Bereich von 4 bis 5. Dementsprechend kann mit diesen Produkten das Histamin auch aus alkoholischen Getränken, die einen sauren pH-Wert aufweisen, wie zum Beispiel Wein, entfernt werden.

Die Erfindung sei nun anhand von Beispiellen näher erläutert.

### Beispiel 1

Herstellung von Histaminase enthaltenden Produkten durch Verwendung von Milchsäurebakterien.

Als Mikroorganismen wurden Mutanten von Lactobacillus delbrueckii oder Lactobacillus leichmannii verwendet. Diese beiden Mikroorganismmenstämme bilden aus Glucose, anderen Zukkern, bzw. anderen Kohlehydraten homofermentativ D(-1)-Milchsäure. Beide Mikroorganismenstämme bilden neben der Histaminase auch noch Transaminasen und Desaminasen. Die Transaminasen und Desaminasen sind nicht geeignet um bereits vorhandenes Histamin abzubauen, sondern Transaminasen und Desaminasen bauen lediglich im Ausgangsmaterial enthaltenes Histidin ohne Bildung von Histamin ab. Es ist in keiner Weise schädlich, wenn das durch die Züchtung der Mikroorganismen gewonnene Endprodukt neben der Histaminase, also der Diaminoxidase, auch noch Transaminasen und Desaminasen enthält. Es muss nur Sorge getragen werden, das eine ausreichende Menge an Keimen gezüchtet wird, damit man ausreichende Mengen des Histaminase enthaltenden Endproduktes erhält.

Für die Züchtung der oben genannten Mutanten von Lactobacillus ist die Lactobacillus-Bouillon nach de Man, Rogosa und Sharp gut geeignet.

Diese Nährlösung besitzt die folgende Zusammensetzung:

| Bestandteil | Menge in g/l |
|---|---|
| Pepton | 10 |
| Fleischextract | 8 |
| Hefeextrakt | 4 |
| Dextrose | 20 |
| Di-Kaliumhdyrogenphosphat | 2 |
| Natriumacetat.3 $H_2O$ | 5 |
| tri-Ammoniumcitrat | 2 |
| Magnesiumsulfat.7 $H_2O$ | 0,2 |
| Mangansulfat.4$H_2O$ | 0,05 |

Als weitere Komponente enthielt dieses Medium das oberflächenaktive Mittel Tween 80 in einer menge von 1 ml/1 Medium.

Der pH-Wert des Mediums wurde auf 6,2 ± 0,1 eingestellt. Anschliessend beimpfte man kräftig mit einem der beiden oben genannten Stämme von Lactobacillus und stellte eine Temperatur von 37°C ein. Man bebrütete 3 Tage lang bei 37°C unter anaeroben Bedingungen unter Zusatz von Kohlendioxidentwicklern. Nach dieser Zeit hatte eine ausreichende Zellvermehrung stattgefunden und die Zellen wurden durch Zentrifugieren mit etwa 20'000 g abgeschleudert und diese Zellmasse wurde sodann sofort auf +4°C gekühlt.

Anschliessend wurde die Biomasse in physiologischer Kochsalzlösung im Verhältnis von einem Gewichtsteil Biomasse zu 10 Gewichtsteilen physiologische Kochsalzlösung resuspendiert. Dann wurde erneut zentrifugiert, und zwar wieder mit etwa 20'000 g. Die so gewonnene Zellmasse wurde fein zerkleinert und gefriergetrocknet. Dabei wurde Sorge getragen, dass das Produkt so lange der Gefriertrocknung unterworfen wurde, bis es wirklich vollständig trocken war.

Das so hergestellte gefriergetrocknete Produkte wurde bei einer Temperatur von nicht über +44°C in absolut trockenem Zustand aufbewahrt. Es zeigte sich, dass das Produkt, nach Einbringung in einen 0,2 molaren Acetat-Puffer eines pH-Wertes von 4,9 in der Lage ist, Histamin volständig abzubauen.

Nach einer Lagerungszeit von mehreren Wochen unter den oben angegebenen Bedingungen hatte das Produkt seine enzymatische Aktivität beibehalten.

### Beispiel 2

Die im Beispiel 1 genannten Lactobacillusstämme wurden in der gleichen Weise gezüchtet, wie im Beispiel 1 und die Zellen wurden, so wie im Beispiel 1 beschrieben, durch Zentrifugieren aus der Kultur isoliert, dann in physiologischer Kochsalzlösung erneut suspendiert und wieder durch Zentrifugieren isoliert.

Die so gewonnenen gereinigten Zellen wurden zerkleinert und auf 4°C gekühlt und bei dieser Temperatur mit 5 Volumina Aceton einer Temperatur von 4°C pro Volumen der zerkleinerten Biomasse vermischt. Es wurde in der Kälte so lange gerührt bis ein Gerinnen eintrat. Man wartete bis sich ein Sediment abgeschieden hatte und heberte dann das überstehende Aceton ab. Der Rückstand wurde in einem Buchner-Trichter gesammelt. Dabei wurde das weitere Absaugen so vorsichtig durchgeführt, dass ein Reissen des Filterkuchens verhindert wurde. Es ist wesentlich, dass ein Reissen des Filterkuchens beim Absaugen vermieden wird, weil anderenfalls die anschliessenden Waschvorgänge mit dem Aceton zu keiner ausreichenden Reinigung des Filterrückstandes führen.

Der Filterkuchen wurde sodann 2x mit Aceton und 1x mit Diäthyläther gewaschen, abgesaugt, zerkleinert und an der Luft vorgetrocknet.

Nach der Vortrocknung an der Luft wurde das Produkt im Vakuum über einem Trocknungsmittel, beispielsweise Schwefelsäure, nachgetrocknet und dann im Exsikkator bei +4°C aufbewahrt.

Eine wirksame Trocknung ist wesentlich, damit die Haltbarkeit des Produktes gewährleistet ist.

### Beispiel 3

Nach dem im Beispiel 1 beschriebenen Verfahren wurde die Mutante von Lactobacillus delbrükkii gezüchet und die Isolierung der Zellen aus dem Zuchtmedium erfolgte ebenfalls in der gleichen Weise wie im Beispiel 1. Auch die Resuspendierung der abgeschleuderten Biomasse in physiologischer Kochsalzlösung und das erneute Abzentrifugieren der Zellen erfolgte in der gleichen Weise, wie im Beispiel 1 beschrieben.

Die so gereinigten Zellen wurden auf 4°C gekühlt. Anschliessend wurden die Mikroorganismenzellen, ebenfalls bei einer Temperatur von 4°C mit Hilfe eines Homogenisators fein zerkleinert.

Zu diesem Zweck wurde ein Homogenisator nach Potter-Elverhjem benutzt. Dieser Homogenisator besitzt ein mechanisch rotierendes Pistill, das mit geringem Spielraum von ca. 0,25 mm in ein zylindrisches Glasgefäss gedrückt wird, sodass die Zellen in dem Spalt zwischen Pistill und Glaswand mechanisch zerrieben werden. Das Pistill kann in einen Rührwerksmotor eingespannt werden, der mit etwa 1000 U/min. rotiert, wobei das zylindrische Glasgefäss gegen den Stempel auf- und niederbewegt wird. Durch diese Vorrichtung wird eine feine Zerkleinerung der Zellen gewährleistet.

Nach der Zerkleinerung der Zellen wird von diesen mit Hilfe einer Pressvorrichtung der Zellsaft abgepresst und dieser wird in einer eisgekühlten Vorlage bei einer Temperatur von +5°C sofort mit Ammonsulfat versetzt, bis eine 25 Gew.-ige Konzentration an Ammoniumsulfat in dem abgepressten Zellsaft erreicht ist. Dabei bildet sich ein Niederschlag, der abzentrifugiert und verworfen wird.

Die über dem Niederschlag stehende Flüssigkeit wird entnommen und ebenfalls bei 5°C mit weiteren Mengen an Ammonsulfat versetzt, bis eine 55 Gew.-%ige Konzentration an Ammonsulfat in dem wässrigen Medium erreicht ist. Der dabei gebildete Niederschlag wird abzentrifugiert, der abzentrifugierte Feststoff im Vakuum sorgfältig getrocknet, vorzugsweise über $P_2O_5$.

Das getrocknete Produkt wird vermahlen und als Pulver trocken bei 4°C aufbewahrt. Dieses Pulver ist auch nach zweimonatiger Lagerungszeit in der Lage, Histamin vollständig abzubauen.

### Beispiel 4

Der im Beispiel 1 verwendete Stamm von Lactobacillus leichmannii wurde in der im Beispiel 1 beschriebenen Weise gezüchtet, die Zellen wurden sodann durch Abzentrifugieren aus dem Kulturmedium abgetrennt und in der im Beispiel 1 beschriebenen Weise in physiologischer Kochsalzlösung erneut suspendiert und durch Zentrifugieren wieder abgetrennt.

Die Zerkleinerung der so erhaltenen Biomasse erfolgte nach dem im Beispiel 3 beschriebenen Verfahren unter Verwendung eines Homogenisators nach Potter-Elverhjem.

Während der Zerkleinerung der Zellen oder nach ihrer Zerkleinerung, setzt man als Extraktionsmittel eine 0,85 Gew.-%ige Natriumchloridlösung zu, vorzugsweise in einer Menge von 1 bis 2 Gewichtsteilen Natriumchloridlösung pro Gewichtsteil zerkleinerter Zellen. Man erhält so eine Rohenzymlösung, die direkt zum Abbau von Histamin in Nahrungsmitteln, getränken und Futtermitteln herangezogen werden kann.

Die so gewonnene Rohenzymlösung kann jedoch durch Säulenchromatographie weitere gereinigt werden, wobei man als Produkt je nach den durchgeführten Chromatographievorgängen entweder die reine Diaminooxidase, also die reine Histaminase erhält oder ein Produkt, das neben der Histaminase noch andere Enzyme enthält, wobei jedoch der Gehalt an Histaminase gegenüber der Rohenzymlösung um ein Mehrfaches erhöht wurde.

Beispiel 5

Herstellung der Histaminase unter Verwendung einer Mischkultur von Baketerien und Hefen.

Es wurde eine Mischkultur aus dem Hefestamm Candida crusei und dem Milchsäurebakterienstamm Lactobacillus bulgaricus verwendet.

Die Züchtung wurde in zwei Gärbehältern durchgeführt. In beiden Gärbehältern wurde als Zuchtmedium Molke verwendet, deren Eiweissgehalt vorher gesenkt worden war oder die vollständig enteiweisst war. Die Molke hatte einen Milchzuckergehalt von 4,1%. Im ersten Gärbehälter wurde zu Beginn der Gärung zu der Molke Hefeautolysat in einer Menge von 0,5% zugesetzt. Im ersten Gärbehälter und auch im zweiten Gärbehälter wurde die Temperatur bei 44°C konstant gehalten. Ds Füllvolumen im ersten Gärbehälter und im zweiten Gärbehälter betrug 60%.

Im ersten Gärbehälter wurde ständig neue Molke zugeführt, und zwar mit einer konstanten Zuführgeschwindigkeit. Im allgemeinen betrug diese Zuführgeschwindigkeit an Molke etwa 5 Volumenprozent pro Stunde, bezogen auf die im ersten Gärbehälter enthaltene Molke.

Im ersten Gärbehälter wuchs die Mischkultur unter begrenzter Luftzufuhr. Die Luftzufuhr im ersten Behälter wurde so einreguliert, dass die Zusammensetzung der Mischkultur im zweiten Gärbehälter konstant gehalten wurde. Im allgemeinen lag diese Luftzufuhr bei 0,30 Volumina bis 0,40 Volumina Luft pro Volumen Nährmedium pro Minute.

Im ersten gärbehälter wurde ferner der pH-Wert durch Zugabe von Ammoniak auf 5,5 konstant gehalten.

Unter den angewandten Züchtungsbedingungen bauen die Milchsäurebakterien den gesamten in der Molke enthaltenen Milchzucke zu Milchsäure ab, und diese Milchsäure dient als Kohlenstoffquelle für die Hefespezies Candida crusei. Diese Hefespezies baut die Milchsäure weiter ab, wobei sich Acetylcoenzym A bildet, das für die Synthese verbraucht wird.

In der ersten Züchtungsphase, die im ersten Gärbehälter abläuft, nehmen die Hefen hauptsächlich die Milchsäure aus dem Medium auf und verwenden das anwesende Hefeautolysat als Stickstoffquelle für ihre Proteinsynthese. Der erste Gärbehälter dient also zur Einleitung des Wachstums.

Im zweiten Gärbehälter findet die hauptsächliche Wachstumsphase statt und dort wird Ammoniak von der Hefe assimiliert und für ihre Proteinsynthese genützt. Ammoniak wird dabei sowohl künstlich zugesetzt, nämlich in den ersten Gärbehälter, um den pH-Wert bei 5,5 konstant zu halten. Ferner wird jedoch auch Ammoniak aus vorhandenem Histamin gebildet, nämlich dadurch, dass die in den Zellen gebildete Histaminase das Histamin unter Bildung von Ammoniak abbaut.

Das Wachstum der Hefen, das mit einer Stickstoffaufnahme verbunden ist, reicht im ersten Gärbehälter aus, um die Zusammensetzung der Mischkultur konstant zu halten. Die mit Hilfe der Amoniakzugabe durchgeführte Einstellung des pH-Wertes auf 5,5 steuert den Prozess so, dass die Zusammensetzung der Mischkultur im ersten Gärbehälter konstant gehalten wird. In dem ersten Gärbehälter wird also im Laufe der Züchtung das Verhältnis der Anzahl der Hefezellen zu der Anzahl der Zellen der Milchsäurebakterien konstant gehalten.

Auch der zweite Gärbehälter ist mit einer Vorrichtung zur Bestimmung des pH-Wertes versehen. In dem zweiten Gärbehälter wird der pH-Wert ebenfalls bei 5,5 konstant gehalten. In diesem Falle jedoch, erfolgt die Konstanthaltung des pH-Wertes nicht durch Zugabe von weiteren Mengen an Ammoniak, sondern durch eine entsprechende Einstellung der Luftzufuhr im ersten Gärbehälter. Durch diese Massnahme wird ein für das Wachstum der Hefe optimales Verhältnis der intermediär gebildeten Produkte Ammoniumlactat zu Milchsäure erreicht.

Dem zweiten Gärbehälter wird kontinuierlich Kulturbrühe des ersten Gärbehälters zugeführt und der zweite Gärbehälter wird optimal belüftet.

Sobald eine ausreichende Menge an Mischkultur zur Verfügung steht, ist es möglich, das Züchtungsverfahren auch in einem einzigen Gärbehälter durchzuführen. Auch hier ist eine kontinuierliche Arbeitsweise bevorzugt, bei der ständig frische Molke zugesetzt und ständig Lactobacillus und Hefe enthaltende Kulturbrühe abgezogen wird.

Bei der Durchführung des Verfahrens in zwei Gärbehältern wird aus dem zweiten Gärbehälter kontinuierlich Mischkultur abgezogen. Die abgezogene Mischkultur wird sofort abzentrifugiert, vorzugsweise nach dem im Beispiel 1 beschriebenen Verfahren.

Die abzentrifugierte Biomasse wrid dann wie im Beispiel 1 beschrieben, in physiologischer Kochsalzlösung erneut suspendiert und dann wieder abzentrifugiert.

Die so gewonnene rohe Biomasse wird entweder nach dem im Beispiel 1 beschriebenen Verfahren lyophylisiert und gelagert oder nach den in den Beispielen 2 bis 4 beschriebenen Verfahren weiter aufgearbeitet.

### Beispiel 6

**Abbau von Histamin in einem Rotwein**

Die Herstellung des Rotweines unter Verwendung des Mostes erfolgte unter üblichen Gärbedingungen. Nach Abschluss der alkoholischen Gärung wurde der gebildete Rotwein von der Hefe abgetrennt, gekühlt und mit dem gemäss Beispiel 1 hergestellten rohen Enzymprodukt in einer Menge von 0,01 g/1 bis 0,5 g/l versetzt. Nach einem gründlichen Durchmischen wurde der auf 4°C gekühlte Wein gegebenenfalls unter Durchrühren so lange belassen, bis kein Histamin mehr feststellbar ist. Dies ist im allgemeinen nach 1 bis 10 Tagen der Fall. Anschliessend wird erneut filtriert, um das zugesetzte Histaminase enthaltende Material zusammen mit dem abgeschiedenen Weinstein wieder zu entfernen.

Ein gelegentliches Rühren während des Histaminabbaues ist vorteilhaft, damit gewährleistet ist, dass der für den Histaminabbau nötige Sauerstoff immer vorhanden ist.

### Beispiel 7

**Herstellung von histaminfreier Frischmilch**

Pasteurisierte Frischmilch wurde auf 4°C gekühlt und anschliessend mit 0,1 g/l des gemäss Beispiel 1 hergestellten rohen Enzymproduktes versetzt. Sie wurde gelegentlich gerührt und im gekühlten Zustand so lange belassen bis kein Histamin mehr nachgewiesen werden konnte. Anschliessend wurde die Frischmilch direkt für den Verkauf abgepackt oder mit Labferment versetzt, um den Käsebruch zu gewinnen. Im zuletzt genannten Fall ist sowohl das Milchkoagulat als auch die Molke frei von Histamin.

### Patentansprüche

1. Verfahren zum Abbau von Histamin in Nahrungsmitteln, getränken und/oder Futtermitteln durch Zusatz eines aus Mikroorganismen gewonnenen D(-)-Aminooxydasepräparates, dadurch gekennzeichnet,

daß als Mikroorganismus ein Lactobacillus eingesetzt wird, der aus Glucose, anderen Zuckern oder anderen Kohlehydraten homofermentativ Milchsäure bildet, und das D(-)-Aminooxydasepräparat mindestens Teile der vom Zuchtmedium befreiten, zerkleinerten mikroorganismen enthält.

2. Verfahren nach Anspruch 1, dadurch gekennzeichnet,

daß der eingesetzte Mikroorganismus Lactobacillus delbrückii, Lactobacillus leichmanii oder Lactobacillus bulgaricus ist.

3. Verfahren zum Abbau von Histamin in Nahrungsmitteln, Getränken und/oder Futtermitteln durch Zusatz eines aus Mikroorganismen gewonnenen D(-) Aminosäurepräparates, dadurch gekennzeichnet,

daß das D(-)- Aminosäurepräparat aus einer Mischkultur aus mindestens einer Lactobacillusspezies und Candida crusei gewonnen wird, wobei unter den angewandten Züchtungsbedingungen der Lactobacillus im Substrat enthaltene Zucker homofermentativ unter Bildung von Milchsäure abbaut, während die eingesetzte Candida crusei die so gebildete Milchsäure abbaut, jedoch die im Nährmedium vorhandenen Zucker nicht angreift und das D(-)-Aminooxydasepräparat mindestens Teile der vom Zuchtmedium befreiten, zerkleinerten Mikroorganismen enthält.

4. Verfahren nach Anspruch 3, dadurch gekennzeichnet,

daß eine Mischkultur aus Lactobacillus bulgaricus und Candida crusei eingesetzt ist und vorzugsweise bei einer Temperatur von 42 bis 46°C und einem pH-Wert von 5,0 bis 5,5 gezüchtet wird.

5. Verfahren nach einem der Ansprüche 1 bis 4, dadurch gekennzeichnet,

daß man die vom Zuchtmedium abgretrennten zerkleinerten Mikroorganismen im Vakuum zu einem haltbaren D(-)-Aminooxydase enthaltenden Enzympräparat trocknet, wobei die Trocknung vorzugsweise bei einer Temperatur von weniger als 7°C vorgenommen wird.

6. Verfahren nach einem der Ansprüche 1 bis 4, dadurch gekennzeichnet,

daß man die Mikroorganismen einer feinen Zerkleinerung unter Verwendung eines Homogenisators unterwirft, die zerkleinerten Mikroorganismen mit einem wässrigen Extraktionsmittel extrahiert oder von den zerkleinerten Mikroorganismen einem wässrigen Press-Saft abpresst, wobei die Behandlung mit dem wässrigen Extraktionsmittel beziehungsweise das Abpressen vorzugsweise bei einer Temperatur von weniger als 7°C vorgenommen wird, und

daß man anschließend den Extrakt oder den abgepressten Press-Saft unter Vakuum trocknet, vorzugsweise bei einer Temperatur von weniger als 7°C.

7. Verfahren nach einem der Ansprüche 1 bis 4, dadurch gekennzeichnet,

daß man die vom Zuchtmedium abgetrennten zerkleinerten Mikroorganismen unter Verwendung eines Homogenisators fein zerkleinert, anschließend einer Extraktion mit einem wässrigen Extraktionsmittel unterwirft, vorzugsweise bei einer Temperatur von weniger als 7°C, sodann den gewonnenen Extrakt der Säulenchromatographie unterwirft und die Fraktion, welche die D(-)-Aminooxydase enthält, unter Vakuum, vorzugsweise bei einer Temperatur von weniger als 7°C trocknet.

8. D(-)-Aminooxydasepräpart zum Abbau von Histamin in Nahrungsmitteln, Getränken und/oder Futtermitteln, dadurch gekennzeichnet,

daß es aus einem Lactobacillus gemäß Anspruch 1 und 2 gewonnen ist und die D(-)-Aminooxydase und mindestens Teile der vom Zuchtmedium befreiten, zerkleinerten Mikroorganismen enthält.

9. D(-)-Aminooxydasepräparat, dadurch gekennzeichnet,

daß es aus einer Mischkultur aus Candida crusei und einem Lactobacillus gemäß Anspruch 1 und 2 gewonnen ist und die (D(-)-Aminoxydase und mindestens Teile der vom Zuchtmedium befreiten, zerkleinerten Mikroorganismen enthält.

10. Präparat nach Anspruch 9, dadurch gekennzeichnet,

daß es aus Candida crusei und mindestens einer Lactobacillusspezies ausgewählt aus Lactobacilli delbrückii, leichmanii und bulgaricus gewonnen ist.

## Revendications

1. Procédé pour dégrader l'histamine dans les denrées alimentaires, boissons et/ou produits fourragers par addition d'une préparation de D(-)-aminoxydase obtenue à partir de micro-organismes, caractérisé par le fait que l'on utilise comme micro-organisme un lactobacille qui, à partir de glucose, d'autres sucres ou d'autres hydrates de carbone, forme de l'acide lactique par homofermentation, et la préparation de D(-)-aminoxydase contient au moins une certaine proportion des micro-organismes fractionnés, débarrassés du milieu de culture.

2. Procédé selon la revendication 1, caractérisé par le fait que le micro-organisme utilisé est choisi parmi Lactobacillus delbrückii, Lactobacillus leichmanii et Lactobacillus bulgaricus.

3. Procédé pour dégrader l'histamine dans les denrées alimentaires, boissons et/ou produits fourragers par addition d'une préparation de D(-)-aminoxydase obtenue à partir de micro-organismes, caractérisé par le fait que la préparation de D(-)-amino-acide est obtenue à partir d'une culture mixtre composée d'au moins une variété de lactobacille et de Candida crusei, le lactobacille, dans les conditions de culture appliquées, dégradant par homofermentation les sucres contenus dans le substrat avec formation d'acide lactique, alors que Candida crusei utilisée dégrade l'acide lactique formé, mais n'attaque pas les sucres présents dans le milieu de culture, et la préparation de D(-)-aminoxydase contient au moins une certaine proportion des micro-organismes fragmentés, débarrassés du milieu de culture.

4. Procédé selon la revendication 3, caractérisé par le fait que l'on utilise une culture mixt composé de Lactobacillus bulgaricus et de Candida crusei et on cultive de préférence à une température comprise entre 42 et 46 degrés C et à un pH 5,0 à 5,5.

5. Procédé selon l'une des revendications 1 à 4, caractérisé par le fait que les micro-organismes fragmentés, séparés du milieu de culture, sont séchés sous vide pour obtenir une préparation d'enzyme durable, contenant de la D(-)-aminoxydase, le séchage étant avantageusement effectué à une température inférieure à 7 degrés C.

6. Procédé selon l'une des revendications 1 à 4, caractérisé par le fait que l'on soumet les micro-organismes à une fragmentation poussée en utilisant un homogénéisateur, on extrait les micro-organismes fragmentés avec un agent d'extraction aqueux des micro-organismes, le traitement avec l'agent d'extraction aqueux ou l'extraction par pression étant effectués de préférence à une température inférieure à 7 degrés C, on sèche ensuite, sous vide, l'extrait ou le jus de pression extrait par pression, de préférence à une température inférieure à 7 degrés C.

7. Procédé selon l'une des revendications 1 à 4, caractérisé par le fait que l'on divise finement, en utilisant un homogénéisateur, les micro-organismes fragmentés, séparés du milieu de culture, on les soumet ensuite à une extraction avec un agent d'extraction aqueux, de préférence à une température inférieure à 7 degrés C, on soumet l'extrait obtenu à une chromographie sur colonne et on sèche, sous vide, la fraction contenant la D(-)-aminoxydase, de préférence à une température inférieure à 7 degrés C.

8. Préparation de D(-)-aminoxydase pour la dégradation d'histamine dans les denrées alimentaires, boissons et/ou produits fourragers, caractérisée par e fait qu'elle est obtenue à partir d'un lactobacille selon les revendications 1 et 2 et contient la D(-)-aminoxydase et au moins une certaine proportion des micro-organismes fractionnés, débarrassés du milieu de culture.

9. Préparation de D(-)-aminoxydase, caractérisée par le fait qu'elle est obtenue à partir d'une culture mixte composée de Candida crusei et d'un lactobacille, conformément aux revendications 1 et 2, et contient au moins une certaine proportion des micro-organismes divisés, débarassés du milieu de culture.

10. Préparation selon la revendication 9, caractérisée par le fait qu'elle est obtenue à partir de Candida Crusei et d'au moins une variété de lactobacille choisie parmi les lactobacilles delbrückii, leichmanii et bulgaricus.

## Claims

1. A method whereby histamine in food, drinks and/or animal feed is decomposed by adding a D(-)-amino oxidase preparation obtained from microorganisms, characterised in that

the microorganism used is a Lactobacillus which forms lactic acid homofermentatively from glucose, other sugars or other carbohydrates and the D(-)-amino oxidase preparation contains at least parts of the microorganism, comminuted and freed from culture medium.

2. A method according to claim 1, characterised in that the microorganism used is Lactobacillus delbrückii, Lactobacillus leichmanii or Lactobacillus bulgaricus.

3. A method whereby histamine in food, drinks and/or animal feed is decomposed by adding a D(-)-amino acid preparation obtained from mikroorganisms, characterised in that

the D(-)-amino acid preparation is obtained from a mixed culture of at least one Lactobacillus species and Candida crusei, the cultivation conditions being made such that the Lactobacillus homofermentatively decomposes the sugar in the substrate and forms lactic acid, whereas the Candida crusei decomposes the thus-formed lactic acid but does not attack the sugar in the nutrient medium, and the D(-)-amino oxidase preparation contains at least parts of the microorganisms, comminuted and freed from culture medium.

4. A method according to claim 3, characterised in that a mixed culture of Lactobacillus bulgaricus and Candida crusei is used and is cultivated preferably at a temperature of 42 to 46°C and a pH of 5.0 to 5.5.

5. A method according to any of claims 1 to 4, characterised in that the comminuted microorganisms separated from the culture medium are dried in vacuo to form a stable enzyme preparation containing D(-)-amino oxidase, and are preferably dried at a temperature below 7°C.

6. A method according to any of claims 1 to 4, characterised in that the microorganisms are finely comminuted using a homogenizer, the comminuted microorganisms are extracted with an aqueous extraction agent or an aqueous juice is expressed from the comminuted microorganisms, treatment with the aqueous extraction agent or the expression process being carried out preferably at a temperature below 7°C, and the extract or the expressed juice is then dried in vacuo, preferably at a temperature below 7°C.

7. A method according to any of claims 1 to 4, characterised in that the comminuted microorganisms separated from the culture medium are finely comminuted using a homogenizer, then extracted with an aqueous extraction agent, preferably at a temperature below 7°C, and then the resulting extract is subjected to column chromatography and the fraction containing the D-(-)-amino oxidase is dried in vacuo, preferably at a temperature below 7°C.

8. A D(-)-amino oxidase preparation for decomposing histamine in food, drinks and/or animal feed, characterised in that it is obtained from a Lactobacillus according to claim 1 or 2 and contains the D-(-)-amino oxidase and at least parts of the microorganisms, comminuted and freed from the culture medium.

9. A D(-)-amino oxidase preparation, characterised in that it is obtained from a mixed culture of Candida crusei and a Lactobacillus according to claim 1 and 2 and contains the D-(-)-amino oxidase and at least parts of the microorganisms, comminuted and freed from culture medium.

10. A preparation according to claim 9, characterised in that it is obtained from Candida crusei and at least one species of Lactobacillus selected from Lactobacilli delbrückii, leichmanii and bulgaricus.